(19) Europäisches Patentamt
European Patent Office
Office européen des brevets

(11) EP 1 169 325 B1

(12) **EUROPEAN PATENT SPECIFICATION**

(45) Date of publication and mention of the grant of the patent:
**21.05.2003 Bulletin 2003/21**

(21) Application number: **00919054.7**

(22) Date of filing: **11.04.2000**

(51) Int Cl.[7]: **C07F 7/18**, C07D 405/00

(86) International application number:
**PCT/GB00/01375**

(87) International publication number:
**WO 00/061588 (19.10.2000 Gazette 2000/42)**

# (54) NOVEL INTERMEDIATE FOR THE SYNTHESIS OF PROSTAGLANDINS

ZWISCHENVERBINDUNG IN DER HERSTELLUNG VON PROSTAGLANDINE

NOUVEAU PRODUIT INTERMEDIAIRE POUR LA SYNTHESE DES PROSTAGLANDINES

(84) Designated Contracting States:
**AT BE CH CY DE DK ES FI FR GB GR IE IT LI LU MC NL PT SE**
Designated Extension States:
**RO**

(30) Priority: **12.04.1999 GB 9908326**

(43) Date of publication of application:
**09.01.2002 Bulletin 2002/02**

(73) Proprietor: **Chirotech Technology Limited Cambridge CB4 0WG (GB)**

(72) Inventors:
- **JACKSON, Philip, Mark, Chirotech Technology Ltd. Cambridge CB4 OWG (GB)**
- **LENNON, Ian, Campbell, Chirotech Technology Ltd. Cambridge CB4 OWG (GB)**

(74) Representative: **Raynor, John**
**W.H. Beck, Greener & Co**
**7 Stone Buildings**
**Lincoln's Inn**
**London WC2A 3SZ (GB)**

(56) References cited:
**EP-A- 0 639 563**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 169 325 B1

## Description

### Field of the Invention

This invention relates to the preparation of a novel crystalline lactone and its use in prostaglandin synthesis.

### Background of the Invention

The synthetic prostaglandin 16-[3-(trifluoromethyl)phenoxy]-17,18,19,20-tetranor $PGF_{2\alpha}$ and its ester derivatives, in particular the isopropyl ester (**2**), are potent drugs for the treatment of glaucoma and ocular hypertension. Optimum therapeutic benefit is achieved when compound (**2**) is used in the form of the dextrorotatory single enantiomer (+)-**2**, as depicted below. For development as a pharmaceutical product, an economically viable route is required for the synthesis of (+)-**2** in quantities of at least 1 kg.

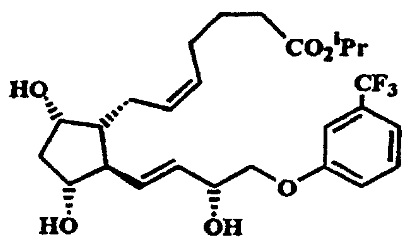

**(2)**

EP-A-0639563 describes biological studies of compound **2** and analogues, and synthetic methods which are applicable to its preparation. The synthetic strategy employed is based on that used by Corey in his pioneering synthesis of prostaglandin $F_{2\alpha}$ (Corey and Cheng, The Logic of Chemical Synthesis, Wiley, 1989, p. 250-266 and references therein), wherein the cyclopentane ring embedded in a lactone intermediate of type **3** (PG = protecting group, e.g. Me) has relative stereochemistry correctly defined across four chiral centres. Lactones of type **3** can be prepared in single enantiomer form. Although such a route was successfully utilised to prepare small quantities of (+)-**2** for preliminary biological evaluation, for a number of reasons it is unsuitable for industrial manufacture as a high-purity pharmaceutical product for administration to human patients. At least 15 steps (from cyclopentadiene) are required, with loss of yield in individual steps exacerbated by the linear nature of the synthesis. Fractional column chromatography is required after many of these steps to effect purification of intermediates. For example, a late stage stereoselective reduction of a 15-keto function in the ω-side chain using (-)-B-chlorodiisopinocampheylborane requires the removal ofthe unwanted 15*S* isomer, formed as a by-product, by a chromatographic separation.

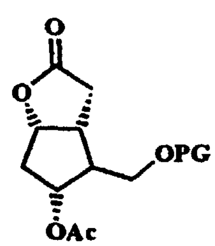

**(3)**

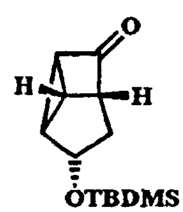

**(4)**

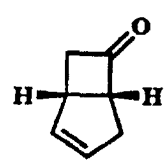

**(5)**

An alternative and more convergent approach to prostaglandins involves addition of a cuprate reagent, incorporating the entire ω-side chain, to the tricyclo[3.2.0.0$^{2,7}$]heptanone **4** (Lee *et al, J. Chem. Soc., Perkin Trans 1,* **1978,** 1176). Tricyclic ketone **4** is prepared from a TBDMS-protected bromohydrin which is in turn derived from bicyclo [3.2.0]hept-2-en-6-one **(5).** In comparison to routes proceeding by a Corey lactone of type **3,** significantly fewer steps are required. For example, preparation of prostaglandin $F_{2\alpha}$ from compound **5** requires only 8 steps (10 steps from cyclopentadiene). Avoidance of awkward late-stage reduction to establish the required configuration of the C-15-OH functionality provides another advantage.

EP-A-0074856 describes resolution of racemic bicyclo[3.2.0]hept-2-en-6-one **(5)** by forming diastereomeric salts of its α-hydroxysulfonic acid derivative with a chiral amine, and separation by crystallisation.

Certain lactones are described in Newton *et al*, Tetrahedron, 1980, 2163. None is crystalline.

Summary of the Invention

This invention is based on the unexpected discovery of a crystalline lactone 1

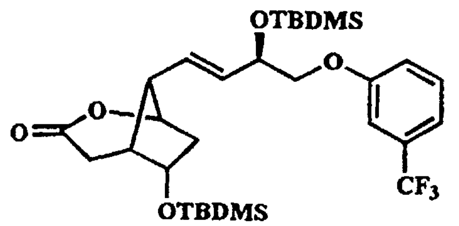

(1)

This compound can be used in the stereoselective synthesis of 16-[3-(trifluoromethyl)phenoxy]-17,18,19,20-tetranor $PGF_{2\alpha}$ and its ester derivatives, for example (+)-**2.** The crystalline lactone can be obtained in highly pure form. The crystallinity of the lactone is crucial in enabling impurities to be removed at this stage without resort to column chromatography. This provides the basis for an industrially viable synthesis of the prostaglandin **2** for pharmaceutical use.

According to the invention, this discovery may also be applied to other substituted 16-phenoxy prostaglandins, where the phenyl substituent may be substituted by one or more haloalkyl, alkyl and halide. The alkyl group may have up to 6 C atoms. Halide is preferably 3-Cl.

Description of the Invention

By way of illustration, the synthesis of the lactone **1** is depicted in Scheme I. All reactants depicted are used in enantiomerically enriched form, typically in >95% ee or higher.

Step (i) is the preparation of the tricyclic ketone **4.** This is achieved by treatment of the bromohydrin **6** with base in an appropriate solvent, preferably potassium *tert*-butoxide in toluene. The unstable tricycle **4** is used without purification in step (iii). It is not necessary to evaporate the tricycle solution to dryness.

Step (ii) is the formation of an alkenylcuprate reagent from the vinyl iodide **7**, precursor to the ω-side chain. The preparation of vinyl iodide **7** in enantiomerically enriched form is disclosed in the copending patent application, filed on the same date, entitled Process for the Preparation of Prostaglandin Precursors, and claiming priority from British Patent Application No. 9908327.1 (WO-0061777). The vinyl iodide is metallated with an alkyllithium reagent, preferably *tert*-butyllithium, and then treated with a cuprate of the form RCu(CN)Li where R is a non-transferable group which may be 2-thienyl. Step (iii) is reaction of the alkenylcuprate with the tricycle to form the bicyclic ketone **8.**

Step (iv) is the Baeyer-Villiger reaction producing the lactone **1**. A peracid, preferably peracetic acid, is used, resulting in a 3:1 mixture of regioisomers, isolated as an oil. Further processing is then required to render this material as usable in subsequent steps. Conveniently, the minor and unwanted regioisomer can be selectively hydrolysed by treatment with aqueous alkali, for example, aqueous sodium hydroxide in acetonitrile. Extraction of the unreacted lactone **1** into an organic solvent, followed by evaporation of solvent, yields a solid residue which can be recrystallised at low temperature to give highly pure crystalline material with convenient handling and storage characteristics. These processing operations are pivotal to the success of the overall synthetic route.

**Scheme I**

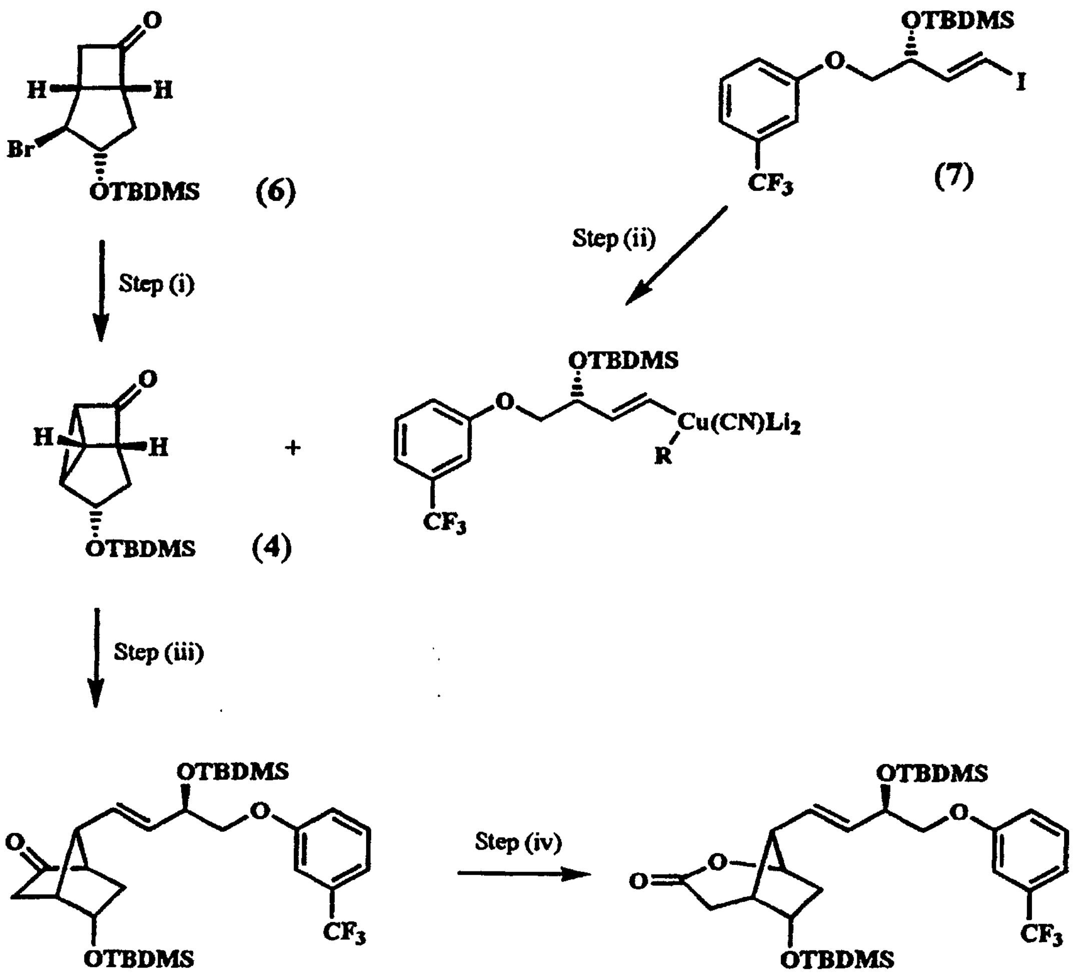


(6) (7) (4) (8) (1)

Scheme II summarises the conversion of lactone **1** to the target prostaglandin (+)-**2,** using conventional processes (for analogous methods see Lee *et al, J. Chem. Soc.*, *Perkin Trans 1*, 1978, 1176; and EP-A-0639563). Typically, reduction to the lactol using diisobutylaluminium hydride is followed by Wittig reaction with the ylide generated from (4-carboxybutyl)triphenylphosphonium bromide and potassium *tert*-butoxide. Esterification and *O*-deprotection steps complete the synthesis.

**Scheme II**

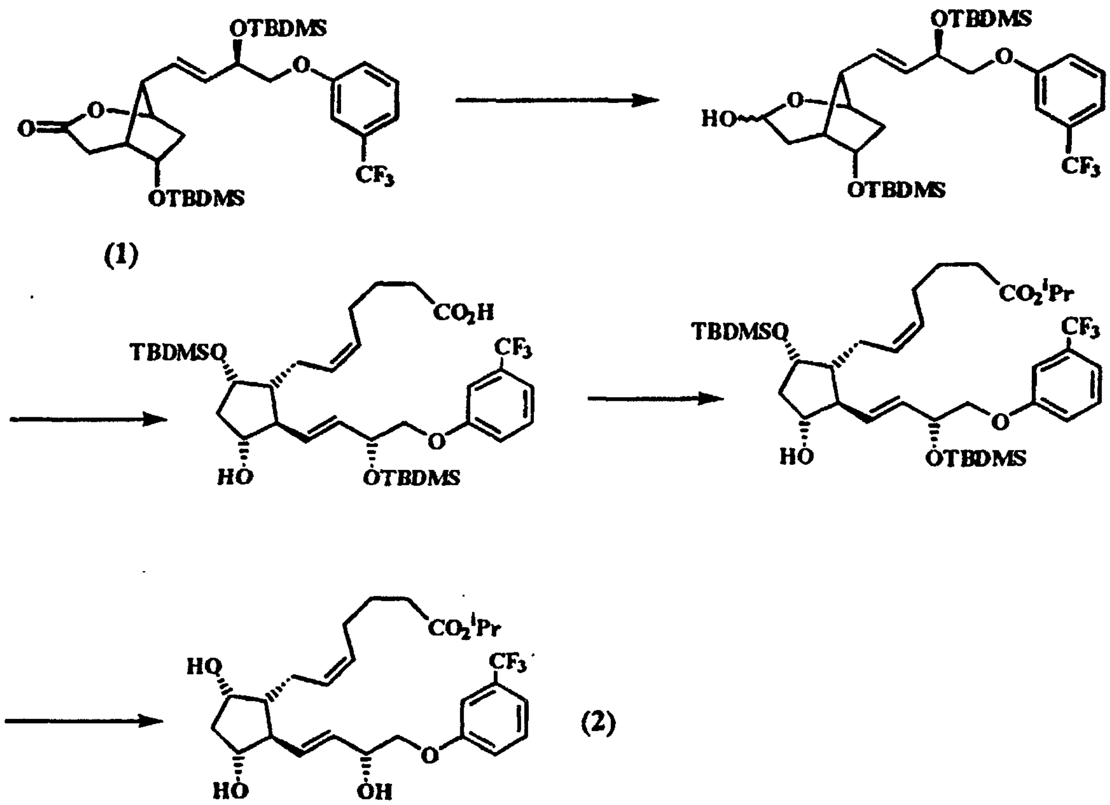

(1)

(2)

The following Examples illustrate the invention.

Example 1 3-*endo*-*tert*-Butyldimethylsilyloxytricyclo[3.2.0.$0^{2,7}$]heptan-6-one

Potassium *tert*-butoxide (114.2 g, 1.02 mol) is suspended in toluene (2 L) and cooled to -15°C. The suspension is stirred under a nitrogen atmosphere and a solution of the bromoketone (250 g, 0.783 mol) in dry toluene (400 ml) is added over 1 hour. The internal temperature is maintained at -10 to -20°C. The mixture is stirred for 1 hour. The mixture is warmed to room temperature, activated carbon (75 g) is added, and the mixture is stirred for 5 minutes. The mixture is filtered through Celite and the cake is washed with toluene (2.5 L). The filtrates are concentrated under reduced pressure, at 20°C, to approximately 700 ml volume. This solution is used directly in the next step.

Example 2 7-*anti*{4-[3-(Trifluoromethyl)phenoxy]-3(*R*)-*tert*-butyldimethylsilyloxy-1(*E*)-butenyl}-5-endo-*tert*-butyldimethylsilyloxybicyclo[2.2.1]hepten-2-one

A dry 10 L flange flask, fitted with an overhead stirrer, temperature probe, nitrogen inlet and pressure equalised dropping funnel, is purged with nitrogen and cooled to -70°C. A solution of *tert*-butyllithium (1.7 M in pentane, 1013 ml, 1.72 mol) is added. The solution is re-cooled to -70°C and a solution of the vinyl iodide (436 g, 0.923 mol) in diethyl ether (1.3 L) is added over 90 minutes, maintaining the internal temperature below -60°C.

In the mean time, thiophene (75.2 ml, 0.94 mol) is placed in a dry 2L 3-necked flask, under a nitrogen atmosphere. THE (600 ml) is added, and the solution is cooled to -30°C. *n*-Butyllithium (2.5 M in hexanes, 376 ml, 0.94 mol) is added over 20 minutes. The solution is stirred for 20 minutes at -20°C, then the resulting yellow solution is added to a suspension of copper(I) cyanide (84.15 g, 0.94 mol) in THF (800 ml) at -20°C over 15 minutes. The resulting dark brown solution is re-cooled to -10°C, and stirred for 20 minutes.

The freshly prepared lithium 2-thienylcyanocuprate solution, at -10°C, is added to the vinyllithium solution at -70°C over 20-30 minutes. The resulting solution is stirred for 30 minutes at -70°C. The tricycle solution (approximately

187 g in 600 ml toluene, + 100 ml THF added) is cooled to -70°C, and added to the cuprate solution , at -70°C over 20 minutes. The mixture is stirred at -70°C for one hour, then the cooling bath is removed from the reaction vessel and saturated ammonium chloride (3 L) is added. The mixture is stirred for 20 minutes, the aqueous layer becomes deep blue in colour and a yellow / green precipitate forms. The mixture is filtered through a No 3 filter paper and the filter cake is washed with methyl *tert*-butyl ether (1 L). The organic layer is separated, and the aqueous layer is extracted with methyl *tert*-butyl ether (1 L). The combined organic layers are washed with brine (2L), dried ($MgSO_4$) and decolourised with activated carbon. After 20 minutes the solution is filtered, the cake is washed with methyl *tert*-butyl ether (2.5 L), and the filtrate is evaporated under reduced pressure.

The residue is taken up in heptane and passed through a plug of silica (1.5 Kg), eluting with 2% EtOAc / heptane to 10% EtOAc / heptane to provide the pure ketone as a yellow solid (293g, 64%), m.p. 64-72°C; $[\alpha]_D^{20}$ +35.9° (c = 1.05, DCM); $\delta_H$ (400 MHz, $CDCl_3$) 7.38 (1 H, t, *J* 8), 7.25 (1 H, d, *J* 8), 7.13 (1 H, s), 7.07 (1 H, d, *J* 8), 5.86 (1 H, dd, *J* 16, 8), 5.73 (1 H, dd, *J* 16, 6), 4.55 (2 H, m), 3.90 (2 H, d, *J* 7), 2.80 (1 H, m), 2.77 (1 H, d, *J* 18), 2.57 (2 H, m), 2.45 (1 H, m), 2.05 (1 H, dd, *J* 18, 4), 1.35 (1 H, m), 0.95 (9 H, s), 0.90 (9 H, s), 0.15 (3 H, s), 0.14 (3 H, s) and 0.05 (6 H, s); $\delta_C$ (100 MHz, $CDCl_3$) 216.0, 158.82, 132.02, 131.54 (q, *J* 32), 129.98, 127.77, 123.90 (q, *J* 270), 118.04, 117.55, 111.13, 72.32, 71.34, 69.92, 54.37, 50.23, 46.24, 38.80, 33.39, 25.76, 18.30, 17.97, -4.67, -4.74, -4.86 and -4.92.

Example 3 8-*anti*{4-[3-(trifluoromethyl)phenoxy]-3(*R*)-*tert*-butyldimethylsilyl-1(*E*)-butenyl}6-*endo-tert*-butyldimethylsilyloxy-2-oxabicyclo[3.2.1]octan-3-one

The ketone (362.6 g, 0.62 mol) and sodium acetate (170 g, 2.07 mol) are dissolved in glacial acetic acid (1.7 L). The reaction vessel is placed in a water bath at 20°C and peracetic acid (40% in dilute acetic acid, 176.7 ml, 0.93 mol) is added over a period of 20 minutes. The solution is stirred at room temperature for 3h. More peracetic acid (30 ml) is added, and the solution is stirred for a further 2h. The reaction mixture is poured onto water (2.5 L), and the products are extracted into MTBE (2 x 750 ml, then 500 ml). The combined organic phases are washed with water (2 L). The aqueous phase is back extracted with MTBE (500 ml). The combined organic extracts are neutralised with saturated sodium carbonate solution (500 ml) and water (2 L) is added to aid phase separation. The organic phase is washed with water (1 L), brine (1 L), dried ($MgSO_4$) and evaporated under reduced pressure, to give a yellow oil (363.8 g). The crude product (consisting of a mixture of regioisomers) is dissolved in acetonitrile (1 L) at room temperature. Sodium hydroxide solution (1M, 300 ml) is added and the solution stirred at room temperature for 2h. Water (1 L) is added and the product is extracted into MTBE (3 X 500 ml). The combined organic phases are washed with brine (500 ml), dried ($MgSO_4$), filtered and evaporated under reduced pressure. The residue is recrystallised from heptane (1 L) at -70°C (cold bath temperature), filtered and washed with cold (-70°C, cold bath) heptane (2 x 200 ml). The solid is dried, to give the lactone as a white solid (141.2 g, 38%). The mother liquors are filtered through silica gel (1 Kg) eluting with 20% DCM / Heptane, to remove baseline. Recrystallisation from cold (-70°C, cold bath) heptane (400 ml) gave a second batch of lactone (34 g, 9%), m.p. 77-78°C; $[\alpha]_D^{20}$ -10.9° (c = 1.05, DCM); $\delta_H$ (400 MHz, $CDCl_3$) 7.40 (1 H, t, *J* 8), 7.22 (1 H, d, *J* 8), 7.10(1 H, s), 7.05 (1 H, d, *J* 8), 5.70 (2 H, m), 4.52 (3 H, m), 3.87 (2 H, d, *J* 6), 3.16 (1 H, d, *J* 18), 3.00 (1H, d, *J* 6), 2.56 (1 H, dd, *J* 18, 6), 2.46 (1 H, m), 2.39 (1 H, m), 1.88 (1 H, dt, *J* 16, 3), 0.95 (9 H, s), 0.89 (9 H, s), 0.10 (6 H, s) and 0.04 (6 H, s); $\delta_C$ (100 MHz, $CDCl_3$) 170.39, 158.75, 132.43, 131.92 (q, *J* 32), 130.05, 127.87, 123.91 (q, *J* 270), 118.06, 117.68, 111.11, 82.20, 72.26, 71.64, 71.10, 48.94, 42.48, 40.40, 33.05, 25.75, 18.30, 18.04, -4.64, -4.76, -4.88 and -5.10.

The lactone of example 7 can be converted to (+)-16-[3-(trifluoromethyl)phenoxy]-17,18,19,20-tetranor $PGF_{2\alpha}$ isopropyl ester using conventional processes; see Scheme II .

## Claims

1. An enantiomerically enriched compound of formula **1**

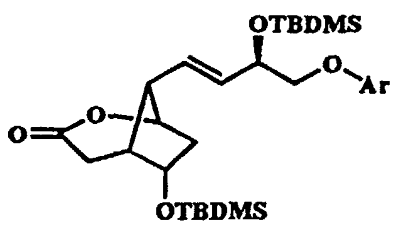

(1)

wherein Ar is phenyl optionally substituted with one or more groups selected from haloalkyl, alkyl and halide.

**2.** A compound according to claim 1, which is 8-*anti*{4-[3-(trifluoromethyl)phenoxy]-3*R*-dimethyl-*tert*-butylsilyloxy-1*E*-butenyl}-6-*endo*-dimethyl-*tert*-butylsilyloxy-2-oxabicyclo[3.2.1]octan-3-one.

**3.** A compound according to claim 1 or claim 2, isolable in crystalline form.

**4.** A process for preparing a compound according to any of claims 1 to 3, which comprises the following steps: reaction of (-)-2-exo-bromo-3-endo-*tert*-butyldimethylsilyloxybicyclo[3.2.0]heptan-6-one with base to form 3-endo-*tert*-butyldimethylsilyloxytricyclo[3.2.0.0$^{2,7}$]heptanone; reaction of the latter compound with a cuprate reagent derived from (*R*)-4-(aryloxy)-3-(*tert*-butyldimethylsilyloxy)- 1-iodo-1*E*-butene or the corresponding alkyne; and Baeyer-Villiger oxidation ofthe resultant bicyclic ketone, with removal of the regioisomeric lactone via hydrolysis.

**5.** A process according to claim 4, wherein aryl is 3-(trifluoromethyl)phenyl, for preparation of a compound according to claim 2.

**6.** Use of a compound of any of claims 1 to 3, for the preparation of a prostaglandin or an ester derivative thereof.

**7.** Use according to claim 6, wherein the prostaglandin is (+)-16-[3-trifluoromethyl)phenoxy]-17,18,19,20-tetranor $PGF_{2\alpha}$ isopropyl ester.

**Patentansprüche**

**1.** Eine enantiomerisch angereicherte Verbindung der Formel 1

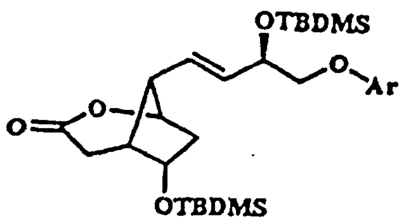

(1)

worin Ar Phenyl ist, gegebenenfalls substituiert mit einer oder mehreren Gruppen, ausgewählt aus Haloalkyl, Alkyl und Halogen.

**2.** Eine Verbindung gemäß Anspruch 1, welche 8-*anti*-{4-[3-(Trifluormethyl)phenoxy]-3*R*-dimethyl-*tert*-butylsilyloxy-1*E*butenyl}-6-*endo*-dimethyl-*tert*-butylsilyloxy-2-oxabicyclo[3.2.1]octan-3-on ist.

**3.** Eine Verbindung gemäß Anspruch 1 oder Anspruch 2, isolierbar in kristalliner Form.

**4.** Ein Verfahren zur Herstellung einer Verbindung gemäß einem der Ansprüche 1 bis 3, welches die folgenden Schritte umfasst:

Reagieren von (-)-2-*exo*-Brom-3-*endo*-*tert*-butyldimethylsilyloxybicyclo[3.2.0]heptan-6-on mit einer Base, um 3-*endo*-*tert*-Butyldimethylsilyloxytricyclo[3.2.0.0$^{2,7}$]heptanon zu bilden,
Reagieren der letztgenannten Verbindung mit einem Cupratreagens, abgeleitet von (*R*)-4-(Aryloxy)-3-(*tert*-butyldimethylsilyloxy)-1-iod-1*E*-buten oder dem entsprechenden Alkin, und
eine Bayer-Villiger-Oxidation des sich ergebenden bicyclischen Ketons mit dem Entfernen des regioisomeren Lactons durch Hydrolyse.

**5.** Ein Verfahren gemäß Anspruch 4, worin das Aryl 3-(Trifluormethyl)phenyl ist, zur Herstellung einer Verbindung gemäß Anspruch 2.

**6.** Verwendung einer Verbindung eines der Ansprüche 1 bis 3 zur Herstellung eines Prostaglandins oder eines Esterderivats davon.

7. Verwendung gemäß Anspruch 6, wobei das Prostaglandin (+)-16-[3-Trifluormethyl)phenoxy]-17,18,19,20-tetranor-$PGF_{2\alpha}$-isopropylester ist.

**Revendications**

1. Composé énantiomériquement enrichi de formule 1

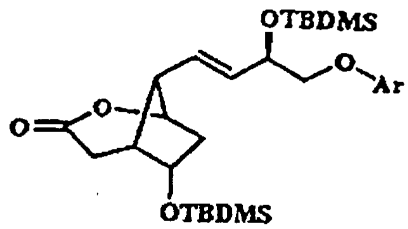

(1)

dans laquelle Ar représente un phényle éventuellement substitué par un ou plusieurs groupes choisis parmi les groupes halogénoalkyle, alkyle et halogénure.

2. Composé selon la revendication 1, qui est le 8-*anti*{4-[3- (trifluorométhyl)phénoxy]-3*R*-diméthyl-*tert*-butylsilyloxy-1*E*-butényl}-6-*endo*-diméthyl-*tert*-butylsilyloxy-2-oxabicyclo[3.2.1]octan-3-one.

3. Composé selon la revendication 1 ou la revendication 2, pouvant être isolé sous forme cristalline.

4. Procédé de préparation d'un composé selon l'une quelconque des revendications 1 à 3 comprenant les étapes suivantes : la réaction de (-)-2-exo-bromo-3-endo-*tert*-butyldiméthylsilyloxybicyclo[3.2.0]heptan-6-one avec une base pour former la 3-endo-*tert*-butyldiméthylsilyloxytricyclo[3.2.0.0$^{2,7}$]heptanone ; la réaction de ce dernier composé avec un réactif de cuprate dérivé du (R)-4-(aryloxy)-3-(*tert*-butyldiméthylsilyloxy)-1-iodo-1*E*-butène ou de l'alcyne correspondant ; et une oxydation de Baeyer-Villiger de la cétone bicyclique résultante, avec élimination de la lactone régioisomère par hydrolyse.

5. Procédé selon la revendication 4, dans lequel l'aryle est un 3-(trifluorométhyl)phényle, servant à la préparation d'un composé selon la revendication 2.

6. Utilisation d'un composé de l'une quelconque des revendications 1 à 3 pour la préparation d'une prostaglandine ou d'un dérivé ester de celle-ci.

7. Utilisation selon la revendication 6, dans laquelle la prostaglandine est un ester isopropylique de $PGF_{2\alpha}$ (+)-16-[3-(trifluorométhyl)phénoxy]-17,18,19,20-tétranor.